**Europäisches Patentamt**

**European Patent Office**

**Office européen des brevets**

(19)

(11)　**EP 0 933 344 B1**

(12)　**FASCICULE DE BREVET EUROPEEN**

(45) Date de publication et mention
de la délivrance du brevet:
**18.09.2002   Bulletin 2002/38**

(51) Int Cl.[7]: **C07C 6/04**

(21) Numéro de dépôt: **98403070.0**

(22) Date de dépôt: **07.12.1998**

(54) **Procédé alterné pour la métathèse des oléfines**

Abwechselndes Verfahren zur Metathese von Olefinen

Alternating process for the metathesis of olefins

(84) Etats contractants désignés:
**DE ES GB IT NL**

(30) Priorité:  **10.12.1997  FR 9715743**

(43) Date de publication de la demande:
**04.08.1999   Bulletin 1999/31**

(73) Titulaire: **INSTITUT FRANCAIS DU PETROLE**
**92852 Rueil-Malmaison Cedex (FR)**

(72) Inventeurs:
　• **Coupard, Vincent**
　　**69003 Lyon (FR)**
　• **Hugues, François**
　　**69390 Vernaison (FR)**
　• **Commereuc, Dominique**
　　**92190 Meudon (FR)**
　• **Fischer, Béatrice**
　　**92500 Rueil Malmaison (FR)**
　• **Boucot, Pierre**
　　**69360 Ternay (FR)**
　• **Forestiere, Alain**
　　**69390 Vernaison (FR)**

(74) Mandataire: **Andréeff, François**
**Département Brevets,**
**Institut Français du Petrole,**
**1 & 4 avenue de Bois-Préau**
**92852 Rueil Malmaison (FR)**

(56) Documents cités:
**CH-A- 298 872**　　　　**FR-A- 1 199 407**
**FR-A- 2 608 595**

## Description

Le domaine de l'invention concerne la production d'oléfines à partir d'au moins une oléfine ayant un nombre de carbone différent de celui de la ou des oléfine(s) recherchée(s). Parmi les procédés qui permettent de réaliser cette réaction, on peut citer les procédés d'oligomérisation et les procédés de métathèse ou de disproportionation des oléfines. Le domaine de l'invention concerne plus particulièrement la métathèse ou la disproportionation des oléfines. La métathèse ou disproportionation des oléfines, ou réaction de redistribution des groupements alkylidènes entre eux, présente un grand intérêt pratique, par exemple pour le rééquilibrage entre elles des oléfines légères issues du craquage à la vapeur d'eau ou du craquage catalytique en lit fluide (FCC), telles que l'éthylène, le propylène et les butènes.

Des procédés de métathèse des oléfines ont déjà été décrit notamment dans les brevets. US 4795734, FR 2608595, US 5449852 et FR 2740056: Dans le document FR 2608595, la réaction est effectuée dans un réacteur à lit mobile de catalyseur, un traitement de régénération du catalyseur est prévu, celui-ci est effectué comme suit : on soutire en continu ou périodiquement une partie du catalyseur, ce catalyseur est envoyé dans un ballon accumulateur puis dans un dispositif de régénération du catalyseur. Le catalyseur régénéré est renvoyé en tête de la zone de réaction. Par ailleurs, le brevet US-A- 3 346 661 concerne la réaction de métathèse correspond à une mise en oeuvre en lit fixe en présence d'un catalyseur à base d'oxyde de molybdène ou de tungstène, généralement à température élevée de l'ordre de 300 à 350°C. La phase de régénération du catalyseur est effectuée alternativement avec la réaction dans le même réacteur.

La présente invention concerne un procédé pour effectuer en continu la réaction de métathèse ou de disproportionation des oléfines comprenant au moins 2 phases, une phase a) de réaction effectuée dans une zone comprenant au moins un réacteur contenant au moins un catalyseur en lit fixe et une phase b) de régénération effectuée dans une zone comprenant au moins un réacteur contenant au moins un catalyseur en lit fixe caractérisé en ce qu'alternativement au moins un réacteur passe d'une phase à l'autre et en ce que le dit réacteur avant la mise en régénération de son catalyseur est isolé du circuit de réaction, les hydrocarbures, au moins en partie liquides contenus dans ce réacteur, étant envoyés dans un ballon tampon et réintroduits dans le dit réacteur après la phase de régénération de son catalyseur.

L'invention concerne aussi un dispositif pour la mise en oeuvre de ce procédé qui comprend une zone réactionelle comprenant au moins 1 réacteur contenant au moins un catalyseur en lit fixe et une zone de régénération contenant au moins un réacteur contenant au moins un lit fixe de catalyseur.

Le dispositif comprend souvent de 2 à 10 réacteurs, de préférence de 2 à 6 réacteurs et de manière encore plus préférée de 4 réacteurs, quand la zone réactionelle comprend au moins 2 réacteurs, ils sont montés en série. La zone de régénération comprend de manière préférée un seul réacteur. En phase de réaction, les réacteurs fonctionnent à courant ascendant, ils peuvent aussi fonctionner à courant descendant ou en mode mixte c'est-à-dire qu'une partie des réacteurs fonctionne à courant ascendant, les autres réacteurs fonctionnant à courant descendant. Un fonctionnement à courant ascendant est cependant préféré. Selon le procédé, chaque réacteur est alternativement en fonctionnement puis en phase de régénération de son catalyseur. Le passage de la phase de fonctionnement d'un réacteur à la phase de régénération de son catalyseur s'effectuant selon la procédure suivante : ce réacteur est isolé du reste du dispositif, les hydrocarbures contenus dans ce réacteur sont ensuite évacués puis le réacteur est purgé. Ledit réacteur est ensuite connecté à une boucle de régénération et soumis à un traitement de régénération de son catalyseur, à la fin de cette phase de régénération, le réacteur et la boucle de régénération sont purgés par au moins un traitement de purge par exemple à l'aide d'un gaz inerte ou par le vide ou successivement par au moins une purge à l'aide d'un gaz inerte puis par le vide. On peut également effectuer une ou plusieurs purges par le vide. Après la régénération du catalyseur le réacteur est replacé dans le train des réacteurs en fonctionnement, de préférence en fin de la série.

Les réacteurs en fonctionnement peuvent être agencés en série dans un ordre quelconque. De préférence, le réacteur contenant le catalyseur le plus âgé est placé en tête (au contact de la charge fraîche), et le réacteur contenant le catalyseur fraîchement régénéré est placé en fin de série. Cet agencement apporte les meilleures performances pour la transformation de la charge.

Suivant un mode préféré de réalisation du procédé selon l'invention, on isole le réacteur à régénérer du circuit de fonctionnement puis on vide les hydrocarbures contenus dans le réacteur à régénérer dans un récipient avant de l'insÈrer dans la boude de régénération.

Ce récipient peut être un ballon tampon prévu à cet effet. Après la phase de régénération du catalyseur, on envoie les hydrocarbures contenus dans ce ballon tampon dans le réacteur dont le catalyseur a été régénéré.

Ce récipient peut aussi être un autre réacteur, la procédure est alors la suivante : le réacteur dont on veut régénérer le catalyseur est isolé du circuit de fonctionnement puis on vide les hydrocarbures contenus dans ce réacteur dans le réacteur qui vient d'être régénéré. Le réacteur dont le catalyseur vient d'être régénéré est replacé dans le circuit de fonctionnement, de préférence en fin de la série des réacteurs, et le réacteur vidé de son contenu est connecté au circuit de régénération.

De manière préférée, on récupère les hydrocarbures contenus dans le réacteur à régénérer dans un ballon tampon.

Parmi les avantages du procédé selon l'invention, on peut noter l'utilisation de plusieurs réacteurs que l'on sort alternativement du circuit de fonctionnement pour permettre la régénération de leur catalyseur. Ainsi, si l'on considère l'ensemble de la masse catalytique (l'ensemble des catalyseurs), l'utilisation de plusieurs réacteurs permet de diminuer la masse catalytique inutilisée, plus exactement la masse catalytique en régénération. Ce fonctionnement permet de réaliser un gain économique important, d'autant plus que certains catalyseurs utilisés -comme les catalyseurs contenant du rhénium- coûtent particulièrement chers. L'utilisation de plusieurs réacteurs rend possible l'emploi de catalyseurs de compositions et de masses différentes et elle permet aussi d'intercaler des éléments (fours, refroidisseurs, pompes, moyens de contrôle de la composition de l'effluent...) selon les besoins spécifiques de la réaction. On connaît différents types de catalyseurs pour la métathèse ou la disproportionation des oléfines qui permettent d'effectuer soit des réactions de type homogène quand les éléments constitutifs sont tous solubles dans le milieu de la réaction, soit de type hétérogène quand au moins un des éléments est insoluble dans ledit milieu. Les catalyseurs utilisés dans la réalisation du procédé selon l'invention sont des catalyseurs solides contenant de préférence au moins du rhénium sur un support poreux contenant de préférence de l'alumine. Les brevets US 4795734, FR 2608595, US 5449852, FR 2740056 décrivent des catalyseurs de ce type.

Les oléfines susceptibles de réagir en métathèse ou en disproportionation catalysée par ce type de catalyseurs à base de rhénium supporté peuvent être des oléfines linéaires ou ramifiées, de préférence linéaires, répondant à la formule générale :

$R_1 R_2 C = C R_3 R_4$ où $R_1$, $R_2$, $R_3$, $R_4$ identiques ou différents, sont l'hydrogène ou un radical hydrocarbyle contenant de 1 à 20 atomes de carbone. Les oléfines peuvent aussi présenter une structure cyclique, le cycle comportant de 3 à 20 atomes de carbone. On peut faire agir une oléfine sur elle-même ou plusieurs oléfines en mélange. Les réactions qui nous intéressent de manière préférée sont la métathèse de l'éthylène et d'une coupe C4 contenant du butène-2 qui produit du propylène, la métathèse de l'éthylène et d'une coupe C5 contenant du pentène-2 qui produit du propylène et des butènes. C'est-à-dire les réactions suivantes :

$$\text{Butène -2 + Ethylène} \rightleftharpoons \text{Propylène}$$

$$\text{Pentène -2 + Ethylène} \rightleftharpoons \text{Propylène + Butène -2 + Butène -1}$$

Le dispositif selon l'invention met en oeuvre de 2 à 10 réacteurs, de préférence 2 à 6 réacteurs et de manière encore plus préférée 4 réacteurs. Le procédé selon l'invention permet une conversion des oléfines en continu, la régénération du ou des catalyseurs d'un réacteur a le plus souvent une durée d'environ 25 à 35 heures.

La charge à traiter contenant les oléfines est introduite dans le premier réacteur où elles subissent une première métathèse ou disproportionation, ce catalyseur usé permet aussi de capter les éventuelles impuretés contenues dans la charge. Dans les réalisations où au moins 2 réacteurs sont en fonctionnement, l'effluent qui sort du premier réacteur est introduit dans le deuxième réacteur où il subit une deuxième métathèse ou disproportionation. L'effluent traverse ainsi tous les réacteurs en fonctionnement, les oléfines sont soumises à des métathèses ou disproportionations dans chaque réacteur puis l'effluent est évacué de la zone de réaction.

Dans chaque réacteur, les conditions de réaction sont les suivantes : une température d'environ 0 °C à 100 °C, de préférence d'environ 30 à 60 °C, une pression suffisante pour maintenir l'effluent au moins en partie sinon en majorité sous forme liquide et une PPH (masse de charge à traiter/ masse de catalyseur/ heure) d'environ 0,4 à 10 $h^{-1}$, de préférence de 0,5 à 3 $h^{-1}$. En outre le faible échauffement, environ 0,5 °C par réacteur, rend inutile la présence d'éléments de refroidissement entre deux réacteurs.

La régénération du catalyseur d'un réacteur s'effectue selon un procédé en plusieurs étapes, chaque nouveau cycle de régénération est mené selon la procédure explicitée ci-après. Le réacteur dont le catalyseur doit être régénéré est isolé du cicuit de réaction, les hydrocarbures contenus dans ce réacteur sont évacués, ce réacteur est ensuite purgé par un gaz inerte sec et propre puis placé dans le circuit de régénération. Par gaz propre au sens de la présente description, on entend que la teneur en impuretés de ce gaz est au plus 10000 ppm en volume, par gaz sec au sens de la présente description, on entend que la teneur en eau de ce gaz est au plus 1000 ppm en volume, de préférence, on emploie un gaz contenant au plus 1000 ppm en volume d'impuretés et 400 ppm en volume d'eau. Par impuretés, au sens de la présente description, on entend essentiellement l'oxygène. Ce gaz est habituellement choisi dans le groupe formé par l'azote, le dioxyde de carbone, l'argon. On ne sortirait pas du cadre de la présente invention en utilisant un mélange d'au moins 2 de ces gaz mais le plus souvent on utilise de l'azote. En fin de régénération, la purge du système réacteur-boucle de régénération est effectuée, ce peut être, par exemple une purge par le vide.

Avant la mise en régénération du catalyseur, les hydrocarbures contenus dans le réacteur dont le catalyseur doit être régénéré sont évacués. L'étape de récupération des hydrocarbures, qui est facultative, peut être effectuée selon au moins 2 façons : par récupération dans un ballon tampon ou par récupération dans le réacteur qui vient d'être régénéré.

Le réacteur dont on doit régénérer le catalyseur est ensuite isolé de la section de vidange puis on effectue une purge au moyen d'un gaz inerte sec et propre afin d'éliminer les éventuels hydrocarbures restés sur le catalyseur. Ce gaz traverse le réacteur selon un mode ascendant ou descendant, la pression absolue à l'intérieur du système est habituellement d'environ de 3 à 60 bar, de préférence d'environ 5 à 10 bar.

Le réacteur est ensuite connecté au circuit de régénération où il subit un traitement en plusieurs étapes.

Suivant une première étape, un gaz de régénération comprenant de l'azote et de l'oxygène est introduit dans le dispositif. Il est envoyé dans une enceinte o_ il est séché de telle façon que sa teneur en eau à la sortie de cette enceinte soit au plus de 1000 ppm en volume et de préférence de 400 ppm en volume. Puis il est introduit dans une enceinte alimentée en air de telle façon que la teneur en oxygène du mélange qui sort de cette enceinte soit d'environ 0,2 à 5 % en volume. Ce mélange de gaz est ensuite chauffé dans une enceinte munie d'un moyen de chauffage puis envoyé dans le réacteur. Le mélange de gaz traverse le réacteur dont le catalyseur est en phase de régénération. La température du gaz à l'intérieur du réacteur est habituellement d'environ 300 à 500 °C et la pression absolue à l'intérieur du système réacteur - boucle de régénération est habituellement d'environ de 3 à 60 bar, de préférence d'environ 5 à 10 bar. Le mélange de gaz qui sort du réacteur contient de l'azote, de l'oxygène et du dioxyde de carbone. Une partie de ce gaz est évacuée du circuit de régénération, le reste est recyclé. Ce gaz recyclé est refroidi par tout moyen de refroidissement, par exemple avec un échangeur de chaleur, séché de telle façon que sa teneur en eau à la sortie de cette enceinte soit au plus de 1000 ppm en volume et de préférence de 400 ppm en volume, puis introduit à nouveau dans le circuit de régénération. Cette première étape de la phase de régénération a habituellement une durée d'environ 1 à 10 heures, de préférence d'environ 1 à 7 heures.

Suivant une deuxième étape, un gaz de régénération comprenant de l'azote et de l'oxygène est introduit dans le dispositif. Il est envoyé dans une enceinte o_ il est séché de telle façon que sa teneur en eau à la sortie de cette enceinte soit au plus de 1000 ppm en volume et de préférence de 400 ppm en volume. Puis il est introduit dans une enceinte alimentée en air de façon telle que la teneur en oxygène du mélange qui sort de cette enceinte soit d'environ 1 à 22 % en volume, de préférence de 3 à 10 % en volume. La teneur en oxygène de ce mélange est de préférence supérieure à celle du gaz employé dans la première étape de régénération. Ce mélange est ensuite chauffé dans une enceinte munie d'un moyen de chauffage puis le mélange de gaz est envoyé dans le réacteur. Le mélange de gaz traverse le réacteur dont le catalyseur est en phase de régénération. La température à l'intérieur du réacteur est habituellement d'environ 400 à 700 °C et la pression absolue à l'intérieur du système réacteur - boude de régénération est habituellement d'environ de 3 à 100 bar, de préférence d'environ 5 à 10 bar. La température de régénération dans cette étape est habituellement au moins égale à celle de la première étape et elle est souvent plus élevée. La pression dans cette étape peut être plus basse ou plus élevée que celle de la première étape, elle est souvent identique dans les 2 étapes. Le mélange de gaz qui sort du réacteur contient de l'azote, de l'oxygène et du dioxyde de carbone. Une partie de ce gaz est évacuée du circuit de régénération, le reste est recyclé. Ce gaz recyclé est refroidi par tout moyen de refroidissement, par exemple avec un échangeur de chaleur, séché de telle façon que sa teneur en eau à la sortie de cette enceinte soit au plus de 1000 ppm en volume et de préférence de 400 ppm en volume. Puis ce gaz est introduit à nouveau dans le circuit de régénération. Cette deuxième étape de la phase de régénération a habituellement une durée d'environ 1 à 10 heures, de préférence d'environ 1 à heures.

Suivant une troisième étape, on effectue une purge par exemple par le vide du système réacteur-boucle de régénération pour évacuer le mélange de gaz dont la teneur en oxygène est habituellement d'environ de 1 à 22 % en volume. La purge a pour but de diminuer la teneur en oxygène du système à une valeur suffisament basse pour éviter au maximum les risques inhérents au contact de l'oxygène et des hydrocarbures dans les conditions de température et de pression de travail du procédé. La purge par le vide du système réacteur-boucle de régénération est effectuée à l'issue d'une phase de refroidissement du contenu du réacteur. Le système peut être refroidi avec tout moyen de refroidissement, par exemple avec un échangeur de chaleur. Cette purge peut être effectuée par différents moyens pour faire le vide, on peut notamment utiliser une pompe à vide à anneau liquide : pompe à palette, pompe à piston réciproque, pompe centrifuge à anneau liquide, on peut aussi utiliser selon un mode préféré du procédé selon l'invention un éjecteur à vapeur.

Cette troisième étape est le plus souvent effectuée en 2 temps successifs.

Dans un premier temps, on ouvre le système, on ramène ainsi la pression dans le système à la pression atmosphérique, l'oxygène présent est ainsi évacué de la boucle de régénération. Dans un deuxième temps, on diminue la pression à l'intérieur de la boucle avec un moyen pour faire le vide décrit ci-dessus, on dépressurise l'intérieur du circuit jusqu'à une pression absolue habituellement d'environ 0,2 à 0,3 bar. Puis on augmente à nouveau la pression à l'intérieur du circuit avec un moyen de compression interne ou externe au dispositif jusqu'à une pression absolue d'environ 6 à 8 bar, ce moyen de compression peut être par exemple un compresseur alternatif à piston ou un compresseur centrifuge. On ne sortirait pas du cadre de la présente invention en effectuant une dépressurisation plus poussée. De même dans le cadre de la présente invention on peut effectuer, entre le premier et le deuxième temps, une ou plusieurs purges par un gaz inerte, l'étape de pressurisation est le plus souvent effectuée en utilisant un gaz inerte tel que par exemple l'un de ceux cités ci-devant ou un mélange d'au moins 2 de ces gaz.

Une seule série de dépressurisation- pressurisation suffit habituellement pour atteindre une spécification en oxygène dans le système réacteur-boucle de 1000 ppm en volume. Au besoin, si on veut obtenir une spécification en oxygène dans le système réacteur-boucle plus faible et par exemple d'environ 5 ppm, on effectue au moins une deuxième purge par le vide.

La purge du système réacteur-boude de régénération peut aussi être effectuée par une série de pressurisations à une pression absolue habituellement d'environ 6 à 8 bar et de dépressurisations à l'atmosphère en utilisant un gaz inerte propre et sec, on utilise habituellement de l'azote, ce gaz doit alors présenter des teneurs en eau et en impuretés présentées ci-devant. Habituellement au moins trois séries de dépressurisations-pressurisations à l'atmosphère sont nécessaires pour atteindre une spécification en oxygène dans le système réacteur-boucle de 1000 ppm en volume. Si on veut obtenir une spécification en oxygène dans le système réacteur-boucle de 5 ppm en volume, on doit habituellement effectuer au moins 5 séries de dépressurisations -pressurisations à l'atmosphère.

Dans le procédé selon l'invention, une purge du système réacteur-boucle de régénération par le vide est un mode préféré.

Avant de reconnecter le réacteur au circuit de réaction, on doit pressuriser ce réacteur. Habituellement, on adapte la pression du réacteur en utilisant l'effluent de sortie du circuit de réaction.

Si on a choisi le dispositif comprenant un ballon tampon, avant de replacer le réacteur dont le catalyseur vient d'être régénéré dans le circuit de fonctionnement, on adapte la pression du réacteur en utilisant l'effluent de la réaction puis on introduit dans ce réacteur les hydrocarbures momentanément placés dans le ballon tampon.

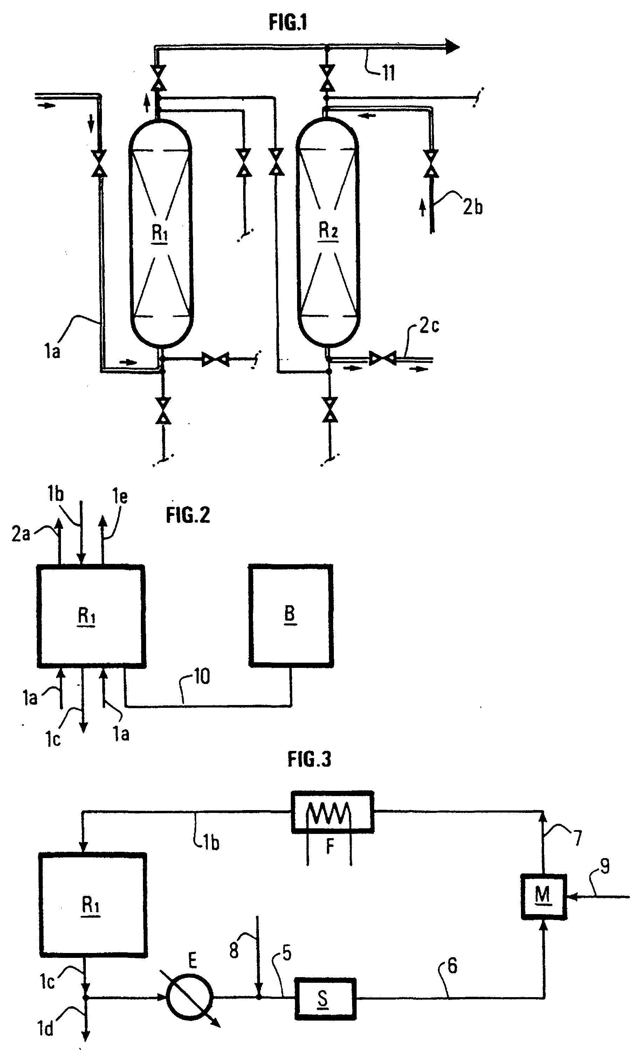

Les figures 1 à 4 illustrent l'invention sans en limiter la portée.

Suivant la figure 1, la charge contenant les oléfines est traitée dans un réacteur. Selon cette figure, l'effluent traverse le réacteur selon un mode à flux ascendant.

La charge à traiter contenant les oléfines est introduite dans le réacteur R1 par la ligne 1a, après avoir réagi, l'effluent sort du circuit par la ligne 11.

Simultanément le réacteur R2 est placé en phase de régénération de son catalyseur, les différents gaz de régénération sont introduits dans le réacteur R2 par la ligne 2b et ressortent de ce réacteur par la ligne 2c.

La figure 2 présente le dispositif de vidange du réacteur avant la mise en régénération de son catalyseur. Dans cette description, on a choisi de présenter la vidange du réacteur R1.

Les lignes 1a et 2a sont respectivement les lignes d'admission de la charge à traiter et d'évacuation de l'effluent produit par le réacteur R1, ces lignes font partie du dispositif de fonctionnement des réacteurs détaillé ci-dessus (figure 1).

Avant la mise en régénération du catalyseur, les hydrocarbures, en majorité liquides, sont introduits dans le ballon B par la ligne 10.

Le réacteur R1 est ensuite isolé de la section de vidange puis on effectue une purge afin d'éliminer les éventuels hydrocarbures restés sur le catalyseur. Le gaz de purge entre dans le réacteur R1 par la ligne 1d et sort du réacteur R1 par la ligne 1e.

A la fin de la phase de régénération du catalyseur du réacteur R1, les hydrocarbures stockés dans le ballon tampon B sont introduits dans le réacteur R1 par la ligne 10.

La figure 3 présente le dispositif de régénération du catalyseur. Dans cette description, on a choisi de présenter la régénération du catalyseur contenu dans le réacteur R1.

Le réacteur dont le catalyseur doit être régénéré est connecté à une boucle de régénération.

Un gaz de régénération est introduit dans le dispositif par la ligne 8, il est ensuite envoyé par la ligne 5 dans une enceinte S munie d'un moyen de séchage puis il est introduit par la ligne 6 dans une enceinte M. L'enceinte M est alimentée en air par la ligne 9. Le mélange sort de l'enceinte M par la ligne 7 et est envoyé dans une enceinte F munie d'un moyen de chauffage puis le mélange est envoyé dans le réacteur R1 par la ligne 1b. Le mélange de gaz traverse le réacteur R1 et sort de ce réacteur par la ligne 1c. Une partie de ce gaz est évacuée du circuit de régénération par la ligne 1d, le reste est refroidi au moyen d'un échangeur de chaleur E puis introduit à nouveau dans le circuit de régénération par la ligne 5.

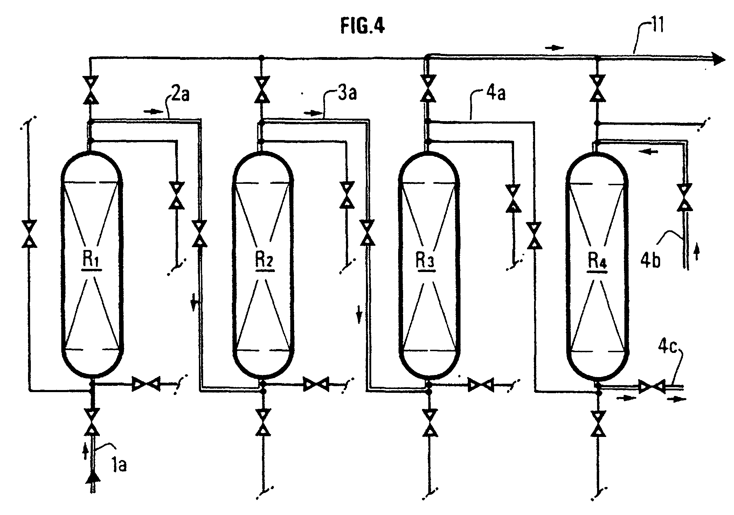

La figure 4 illustre un mode préféré de réalisation du dispositif selon l'invention. Selon ce mode de réalisation, la zone de réaction comprend 3 réacteurs en série, l'effluent parcours successivement les réacteurs R1-R2-R3 dans cet ordre, selon un mode à flux ascendant. La charge à traiter est introduite dans le réacteur R1 par la ligne 1a. Cette charge traverse le réacteur R1 puis ressort par la ligne 2a avant d'être introduite dans le réacteur R2. Cette charge traverse le réacteur R2 puis ressort par la ligne 3a avant d'être introduite dans le réacteur R3. Cette charge traverse le réacteur R3 puis elle est évacuée du circuit par la ligne 11.

Simultanément le réacteur R4 est placé en phase de régénération de son catalyseur, les différents gaz de régénération sont introduits dans le réacteur R4 par la ligne 4b et ressortent de ce réacteur par la ligne 4c.

Les exemples présentés ci-après illustrent l'invention sans en limiter la portée.

Pour des raisons de commodité de mise en oeuvre au laboratoire, les exemples suivants ont été réalisés avec 2 réacteurs dont un en fonctionnement. Cependant les réalisations industrielles comprennent généralement un nombre plus important de réacteurs en fonctionnement, de façon préférée, on utilisera un dispositif comprenant 3 réacteurs en fonctionnement et un seul réacteur en régénération, ainsi qu'un ballon tampon.

Exemples

Les réactions de métathèse des deux exemples qui suivent sont réalisées avec un dispositif selon l'invention comprenant deux réacteurs de volume 5 litres chacun, un est en phase de réaction pendant que l'autre est en phase de régénération. Dans chacun de ces réacteurs, on place 1 kg de catalyseur, le catalyseur utilisé est décrit dans l'exemple 1 du brevet US 4795734. Les réacteurs sont reliés à une boucle de régénération comprenant un refroidisseur, un compresseur, un système d'extraction d'eau et un four. Durant la phase de régénération, une enceinte munie d'une entrée d'air est introduite dans ce dispositif, puis en fin de cette phase de régénération on introduit dans le dispositif une pompe à vide pour faire le vide dans le système.

**exemple 1: métathèse de l'éthylène et du butène-2**

Durant la phase de réaction, un mélange contenant des butènes, principalement du butène-2 est introduit au débit de 0,9 kg/h dans un réacteur avec de l'éthylène pur (qualité polymérisation) au débit de 0,34 kg/h. La réaction est effectuée dans les conditions opératoires suivantes : une température de 35°C et une pression absolue de 35 bar. La composition du mélange à traiter est consignée dans le tableau 1.

Tableau 1:

| Composés | % en poids |
|---|---|
| contenant 3 atomes de carbone ou moins | 0,5 |
| n-butane + i-butane | 15 |
| isobutène | 0,5 |
| butène 1 | 7,5 |
| butène 2 | 75,5 |
| contenant 5 atomes de carbone ou plus | 1 |

La conversion du butène-2 est de 61% en masse en sortie du réacteur. La sélectivité en propylène est de 95% en masse.

Après 30 heures de fonctionnement, on vide le contenu du réacteur qui était en fonctionnement dans le réacteur dont le catalyseur vient d'être régénéré et on place ce dernier réacteur dans le circuit de fonctionnement. Le réacteur dont le catalyseur est usé est placé dans le circuit de régénération.

Durant la phase de régénération qui dure 30 h, un gaz chaud contenant de l'azote, de l'oxygène et du dioxyde de carbone circule sur le catalyseur. Au début de la phase de régénération, la teneur en oxygène de ce mélange est de 0,6% en volume et la teneur en azote sec et propre de 99,4 % en volume, la température à l'intérieur du réacteur est de 450°C et la pression absolue du système boucle-réacteur de 6 bar. Cette étape dure 4 heures. On brûle ainsi d'abord les impuretés déposées lors de la réaction avec un gaz pauvre en oxygène, puis on calcine le catalyseur sous air. Cette calcination est effectuée dans les conditions suivantes : la température à l'intérieur du réacteur est de 550°C et la pression absolue du système boucle-réacteur de 6 bar. A l'issu de cette calcination, qui dure 2 heures, la teneur en oxygène du système boucle-réacteur est de 5 % en volume, la teneur en azote est de 70 % en volume, la teneur en dioxyde de carbone est de 25% en volume. On refroidit la boucle et l'air de combustion qui y circule au moyen d'un échangeur de chaleur, puis on effectue une purge par le vide -on diminue la pression jusqu'à une pression absolue de 0,2 bar- et on remplit ensuite ladite boucle avec de l'azote de telle façon qu'il ne reste dans ladite boude que 0,01 % en volume d'oxygène. On augmente ensuite la pression dans le système jusqu'à une pression absolue de 7 bar avec de l'azote sec et propre contenant 50 ppm en volume d'eau et 300 ppm en volume d'impuretés.

La régénération du catalyseur dure 30 heures, cette durée comprend les périodes de chauffage et de refroidissement. Au bout de 30 heures, le réacteur qui contient le catalyseur qui vient d'être régénéré est replacé dans la zone de réaction et le réacteur dont le catalyseur est usé est placé dans le circuit de régénération.

**Exemple 2 : métathèse de l'éthylène et du pentène-2**

Durant la phase de réaction, un mélange contenant du pentène-2 est introduit au débit de 743 g/h dans un réacteur avec de l'éthylène pur (qualité polymérisation) au débit de 77 g/h. La réaction est effectuée dans les conditions opératoires suivantes : une température de 35°C et une pression absolue de 35 bar. La composition du mélange à traiter est consignée dans le tableau 2.

Tableau 2 :

| Composés | % en poids |
|---|---|
| i-pentane + n-pentane | 63,7 |
| Méthyl 2 butène 2 | 8,3 |
| Pentène 1 | 1,6 |
| Pentène 2 | 15,8 |
| autres oléfines à 5 atomes de carbone ou plus | 10,6 |

La conversion du pentène-2 en sortie du réacteur est de 65% en poids, celle du méthyl-2 butène-2 et des autres oléfines à 5 atomes de carbone ou plus est de 80 % en poids. La sélectivité en oléfines à 3 et 4 atomes de carbone par rapport à l'ensemble des produits formés est de 65% en poids.

Après 30 heures de fonctionnement, on vide le contenu du réacteur en fonctionnement dans le réacteur dont le catalyseur vient d'être régénéré et on place ce dernier réacteur dans le circuit de fonctionnement. Le réacteur dont le catalyseur est usé est placé dans le circuit de régénération.

Cette phase de régénération du catalyseur usé est identique en tous points à celle décrite dans l'exemple 1. A la fin de la régénération du catalyseur, on replace le réacteur contenant le catalyseur régénéré dans la zone de réaction et l'autre réacteur, dont le catalyseur est usé, est placé dans la zone de régénération.

**Revendications**

1. Procédé pour effectuer en continu la réaction de métathèse ou de disproportionation des oléfines comprenant au moins deux phases, une phase a) de réaction effectuée dans une zone comprenant au moins un réacteur contenant au moins un catalyseur en lit fixe et une phase b) de régénération effectuée dans une zone comportant au moins un réacteur contenant au moins un catalyseur en lit fixe, **caractérisé en ce qu'**alternativement au moins un réacteur passe d'une phase à l'autre et **en ce que** le dit réacteur avant la mise en régénération de son catalyseur est isolé du circuit de réaction, les hydrocarbures, au moins en partie liquides contenus dans ce réacteur, étant envoyés dans un ballon tampon et réintroduits dans le dit réacteur après la phase de régénération de son catalyseur.

2. Procédé pour effectuer en continu la réaction de métathèse ou de disproportionation des oléfines selon la revendication 1 dans lequel la phase de réaction est effectuée dans une zone contenant au moins 2 réacteurs en série.

3. Procédé pour effectuer en continu la réaction de métathèse ou de disproportionation des oléfines selon la revendication 1 ou 2 dans lequel la phase de régénération est effectuée dans une zone contenant un seul réacteur.

4. Procédé pour effectuer en continu la réaction de métathèse ou de disproportionation des oléfines selon l'une des revendications 1 à 3 **caractérisé en ce que** le passage de la phase de fonctionnement d'un réacteur à la phase de régénération de son catalyseur s'effectue selon la procédure suivante : le réacteur dont le catalyseur doit être régénéré est isolé du reste du dispositif, les hydrocarbures contenus dans ce réacteur sont évacués, puis ce réacteur est purgé.

5. Procédé pour effectuer en continu la réaction de métathèse ou de disproportionation des oléfines selon l'une des revendications 1 à 4 **caractérisé en ce que** le réacteur dont le catalyseur vient d'être régénéré et la boucle de régénération sont purgés, ledit réacteur étant ensuite replacé dans le train des réacteurs en fonctionnement.

6. Procédé pour effectuer en continu la réaction de métathèse ou de disproportionation des oléfines selon l'une des revendications 1 à 5 **caractérisé en ce qu'**avant avant la mise en régénération du catalyseur d'un réacteur, ledit réacteur est isolé du circuit de réaction, puis les hydrocarbures contenus dans ce réacteur sont vidés dans un autre réacteur, de préférence dans le réacteur dont le catalyseur vient d'être régénéré.

7. Procédé pour effectuer en continu la réaction de métathèse ou de disproportionation des oléfines selon l'une des revendications 1 à 6 **caractérisé en ce que** le réacteur dont le catalyseur vient d'être régénéré est replacé dans le train des réacteurs en fonctionnement, en fin de cette série.

8. Procédé pour effectuer en continu la réaction de métathèse ou de disproportionation des oléfines selon l'une des revendications 1 à 7 **caractérisé en ce que** la phase de régénération du catalyseur est terminée par au moins une dépressurisation du système réacteur-boucle de régénération, la pression du système étant portée à une pression absolue d'environ 0,2 à 0,3 bar, suivie par au moins une pressurisation du système à une pression absolue d'environ 6 à 8 bar.

9. Procédé pour effectuer en continu la réaction de métathèse ou de disproportionation des oléfines selon l'une des revendications 1 à 8 **caractérisé en ce qu'**on fait réagir une coupe C4 contenant du butène-2 et de l'éthylène.

10. Procédé pour effectuer en continu la réaction de métathèse ou de disproportionation des oléfines selon l'une des revendications 1 à 8 **caractérisé en ce qu'**on fait réagir une coupe C5 contenant du pentène-2 et de l'éthylène.

11. Procédé pour effectuer en continu la réaction de métathèse ou de disproportionation des oléfines selon l'une des revendications 1 à 10 **caractérisé en ce que** les réacteurs fonctionnent en mode ascendant.

12. Procédé pour effectuer en continu la réaction de métathèse ou de disproportionation des oléfines selon l'une des revendications 1 à 10 **caractérisé en ce que** les réacteurs fonctionnent en mode descendant.

13. Procédé pour effectuer en continu la réaction de métathèse ou de disproportionation des oléfines selon l'une des revendications 1 à 10 **caractérisé en ce qu'**une partie des réacteurs fonctionne en mode ascendant, les autres réacteurs fonctionnant en mode descendant.

14. Procédé pour effectuer en continu la réaction de métathèse ou de disproportionation des oléfines selon l'une des revendications 1 à 13 **caractérisé en ce que** les catalyseurs utilisés sont des catalyseurs solides contenant au moins du rhénium sur un support poreux contenant de l'alumine.

15. Procédé selon l'une des revendications 1 à 14, dans lequel la température de la zone réactionnelle est de 0 à 100°C et de préférence 30 à 60°C.

16. Dispositif pour la mise en oeuvre du procédé selon l'une des revendications 1 à 15 **caractérisé en ce qu'**il comprend une zone réactionnelle comprenant au moins un réacteur contenant au moins un catalyseur en lit fixe, et une zone de régénération comprenant au moins un réacteur contenant au moins un catalyseur en lit fixe et **en ce qu'**un ballon tampon est disposé en parallèle à la zone réactionnelle.

17. Dispositif pour la mise en oeuvre du procédé selon la revendication 16 **caractérisé en ce que** la zone réactionelle comprend au moins deux réacteurs en série.

## Claims

1. A process for continuous metathesis or disproportionation of olefins, comprising at least two phases, a reaction phase a) carried out in a zone comprising at least one reactor containing at least one fixed bed of catalyst and a regeneration phase b) carried out in a zone comprising at least one reactor containing at least one fixed bed of catalyst, **characterized in that** at least one reactor passes from one phase to the other in alternation and **in that** said reactor, before the passage to regeneration of the catalyst therein, is isolated from the reaction circuit, the hydrocarbons at least partially liquid contained in this reactor being sent to a surge drum and reintroduced into said reactor after the regeneration phase of its catalyst.

2. A process for continuous metathesis or disproportionation of olefins according to claim 1, in which the reaction phase is carried out in a zone containing at least two reactors in series.

3. A process for continuous metathesis or disproportionation of olefins according to claim 1 or claim 2, in which the regeneration phase is carried out in a zone containing a single reactor.

4. A process for continuous metathesis or disproportionation of olefins according to any one of claims 1 to 3, **characterized in that** passage from the operating phase of one reactor to the regeneration phase for its catalyst is accomplished as follows: the reactor for which the catalyst is to be regenerated is isolated from the remainder of the apparatus, the hydrocarbons contained **in that** reactor are evacuated, then said reactor is purged.

5. A process for continuous metathesis or disproportionation of olefins according to any one of claims 1 to 4, **characterized in that** the reactor for which the catalyst has just been regenerated and the regeneration loop are purged, said reactor then being replaced in the series of operating reactors.

6. A process for continuous metathesis or disproportionation of olefins according to any one of claims 1 to 5, **characterized in that** before regenerating the catalyst in a reactor, said reactor is isolated from the reaction circuit, then the hydrocarbons contained in the reactor are emptied into a further reactor, preferably into the reactor for which the catalyst has just been regenerated.

7. A process for continuous metathesis or disproportionation of olefins according to any one of claims 1 to 5, **characterized in that** before regenerating the catalyst of a reactor, said reactor is isolated from the reaction circuit then the hydrocarbons contained **in that** reactor are emptied into a surge drum, and after regeneration of its catalyst, the hydrocarbons in the drum are re-introduced into said reactor, the reactor then being replaced in the series of operating reactors.

8. A process for continuous metathesis or disproportionation of olefins according to any one of claims 1 to 7, **characterized in that** the reactor for which the catalyst has just been regenerated is replaced in the series of operating reactors, at the end of that series.

9. A process for continuous metathesis or disproportionation of olefins according to any one of claims 1 to 8, **characterized in that** the catalyst regeneration phase is terminated by at least one depressurisation step for the reactor-regeneration loop system, the pressure of the system being taken to an absolute pressure of 0.2 to 0.3 bars, followed by at least one pressurisation step for the system to an absolute pressure of about 6 to 8 bars.

10. A process for continuous metathesis or disproportionation of olefins according to any one of claims 1 to 9, **characterized in that** a C4 cut containing 2-butene and ethylene is reacted.

11. A process for continuous metathesis or disproportionation of olefins according to any one of claims 1 to 9, **characterized in that** a C5 cut containing 2-pentene and ethylene is reacted.

12. A process for continuous metathesis or disproportionation of olefins according to any one of claims 1 to 11, **characterized in that** the reactors operate in riser mode.

13. A process for continuous metathesis or disproportionation of olefins according to any one of claims 1 to 11, **characterized in that** the reactors operate in dropper mode.

14. A process for continuous metathesis or disproportionation of olefins according to any one of claims 1 to 11, **characterized in that** a portion of the reactors operate in riser mode, and the other reactors operate in dropper mode.

15. A process for continuous metathesis or disproportionation of olefins according to any one of claims 1 to 14, **characterized in that** the catalysts used are solid catalysts containing at least rhenium on a porous alumina-containing support.

16. An apparatus for carrying out the process claimed in any one of claims 1 to 15, **characterized in that** it comprises a reaction zone comprising at least one reactor containing at least one fixed bed of catalyst and a regeneration zone containing at least one reactor containing at least one fixed bed of catalyst.

17. An apparatus for carrying out the process of claim 16, **characterized in that** the reaction zone comprises at least two reactors in series.

**Patentansprüche**

1. Verfahren zur kontinuierlichen Durchführung der Metathesis- oder Disproportionisierungsreaktion der Olefine, welches wenigstens zwei Phasen, eine Phase a) der Reaktion, durchgeführt in einer Zone, die wenigstens einen Reaktor aufweist, der wenigstens einen Katalysator im festen Bett enthält sowie eine Phase b) der Regenerierung umfasst, die in einer Zone durchgeführt wird, die wenigstens einen Reaktor umfasst, der wenigstens einen Katalysator im festen Bett enthält, **dadurch gekennzeichnet, dass** alternativ wenigstens ein Reaktor von einer Phase zur anderen übergeht und dass dieser Reaktor, bevor sein Katalysator auf Regenerierung gestellt wird, gegenüber dem Reaktionskreis isoliert wird, wobei die wenigstens zum Teil flüssigen in diesem Reaktor enthaltenen Kohlenwasserstoffe in einen Pufferballon gegeben und in diesen Reaktor nach der Regenerierungsphase seines Katalysators wieder eingeführt werden.

2. Verfahren zur kontinuierlichen Durchführung der Metathesis- oder Disproportionierungsreaktion der Olefine gemäß Anspruch 1, bei dem die Reaktionsphase in einer Zone durchgeführt wird, die wenigstens zwei in Reihe geschaltete Reaktoren enthält.

3. Verfahren zur kontinuierlichen Durchführung der Metathesis- oder Disproportionierungsreaktion der Olefine gemäß dem Anspruch 1 oder 2, bei dem die Regenerierungsphase in einer einen einzigen Reaktor enthaltenden Zone durchgeführt wird.

4. Verfahren zur kontinuierlichen Durchführung der Metathesis- oder Disproportionierungsreaktion der Olefine gemäß einem der Ansprüche 1 bis 3, **dadurch gekennzeichnet, dass** der Übergang von der Arbeitsphase eines Reaktors zur Regenerierungsphase seines Katalysators gemäß dem folgenden Ablauf erfolgt: der Reaktor, dessen Katalysator regeneriert werden soll, wird gegenüber dem Rest der Vorrichtung isoliert, die in diesem Reaktor enthaltenen Kohlenwasserstoffe werden abgezogen, dann wird dieser Reaktor gereinigt.

5. Verfahren zur kontinuierlichen Durchführung der Metathesis- oder Disproportionierungsreaktion der Olefine gemäß einem der Ansprüche 1 bis 4, **dadurch gekennzeichnet, dass** der Reaktor, dessen Katalysator gerade regeneriert wurde sowie die Regenerierungsschleife gereinigt werden, wobei dieser Reaktor anschließend in den Zug der im Betrieb befindlichen Reaktoren wieder eingebracht wird.

6. Verfahren zur kontinuierlichen Durchführung der Metathesis- oder Disproportionierungsreaktion der Olefine gemäß einem der Ansprüche 1 bis 5, **dadurch gekennzeichnet, dass** bevor der Katalysator eines Reaktors auf Regeneration gestellt wird, dieser Reaktor gegenüber dem Reaktionskreis isoliert wird, dann die in diesem Reaktor enthaltenen Kohlenwasserstoffe in einen anderen Reaktor, bevorzugt in den Reaktor, dessen Katalysator gerade regeneriert wurde, entleert werden.

7. Verfahren zur kontinuierlichen Durchführung der Metathesis- oder Disproportionierungsreaktion der Olefine gemäß einem der Ansprüche 1 bis 6, **dadurch gekennzeichnet, dass** der Reaktor, dessen Katalysator gerade regeneriert wurde, in den Zug der im Betrieb befindlichen Reaktoren am Ende dieser Reihe wieder eingebracht wird.

8. Verfahren zur kontinuierlichen Durchführung der Metathesis- oder Disproportionierungsreaktion der Olefine gemäß einem der Ansprüche 1 bis 7, **dadurch gekennzeichnet, dass** die Regenerierungsphase des Katalysators durch wenigstens eine Druckentlastung des Systems Reaktor-Regenerierungsschleife beendet wird, wobei der Druck des Systems auf einen Absolutdruck von etwa 0,2 bis 0,3 bar, gefolgt von wenigstens einer Druckentlastung des Systems auf einen Absolutdruck von etwa 6 bis 8 bar gebracht wird.

9. Verfahren zur kontinuierlichen Durchführung der Metathesis- oder Disproportionierungsreaktion der Olefine gemäß einem der Ansprüche 1 bis 8, **dadurch gekennzeichnet, dass** man einen 2-Buten und Ethylen enthaltenden C4-Schnitt reagieren lässt.

10. Verfahren zur kontinuierlichen Durchführung der Metathesis- oder Disproportionierungsreaktion der Olefine gemäß einem der Ansprüche 1 bis 8, **dadurch gekennzeichnet, dass** man einen 2-Penten und Ethylen enthaltenden C5-Schnitt reagieren lässt.

11. Verfahren zur kontinuierlichen Durchführung der Metathesis- oder Disproportionierungsreaktion der Olefine gemäß einem der Ansprüche 1 bis 10, **dadurch gekennzeichnet, dass** die Reaktoren im aufsteigenden Mode betrieben werden.

**12.** Verfahren zur kontinuierlichen Durchführung der Metathesis- oder Disproportionierungsreaktion der Olefine gemäß einem der Ansprüche 1 bis 10, **dadurch gekennzeichnet, dass** die Reaktoren im absteigenden Mode betrieben werden.

**13.** Verfahren zur kontinuierlichen Durchführung der Metathesis- oder Disproportionierungsreaktion der Olefine gemäß einem der Ansprüche 1 bis 10, **dadurch gekennzeichnet, dass** ein Teil der Reaktoren im aufsteigenden Mode betrieben wird, die anderen Reaktoren im absteigenden Mode betrieben werden.

**14.** Verfahren zur kontinuierlichen Durchführung der Metathesis- oder Disproportionierungsreaktion der Olefine gemäß einem der Ansprüche 1 bis 13, **dadurch gekennzeichnet, dass** die verwendeten Katalysatoren feste Katalysatoren sind, die wenigstens Rhenium auf einem porösen Aluminiumoxid enthaltenden Träger enthalten.

**15.** Verfahren nach einem der Ansprüche 1 bis 14, bei dem die Temperatur der Reaktionszone bei 0 bis 100°C, bevorzugt bei 30 bis 60°C, liegt.

**16.** Vorrichtung zur Durchführung des Verfahrens gemäß einem der Ansprüche 1 bis 15, **dadurch gekennzeichnet, dass** sie eine Reaktionszone, die wenigstens einen Reaktor der wenigstens einen Katalysator im festen Bett enthält sowie eine Regenerierungszone umfasst, die wenigstens einen Reaktor umfasst, der wenigstens einen Katalysator im festen Bett enthält und dass ein Pufferballon parallel zur Reaktionszone angeordnet ist.

**17.** Vorrichtung zur Durchführung des Verfahrens nach Anspruch 16, **dadurch gekennzeichnet, dass** die Reaktionszone wenigstens zwei Reaktoren in Reihe umfasst.

FIG.1

11

2b

R₁

R₂

1a

2c

FIG.2

1b

1e

2a

R₁

B

1a

10

1a

1c

FIG.3

1b

F

7

9

M

R₁

E

8

5

6

1c

S

1d

# FIG.4